**Europäisches Patentamt**

**European Patent Office**

**Office européen des brevets**

(19)

(11) Veröffentlichungsnummer : **0 052 858 B2**

(12)

# NEUE EUROPÄISCHE PATENTSCHRIFT

(45) Veröffentlichungstag der neuen Patentschrift :
**21.10.92 Patentblatt 92/43**

(21) Anmeldenummer : **81109752.6**

(22) Anmeldetag : **19.11.81**

(51) Int. Cl.⁵ : **A61F 2/26**

---

(54) **Penisprothese.**

---

(30) Priorität : **24.11.80 DE 8031230 U**

(43) Veröffentlichungstag der Anmeldung :
**02.06.82 Patentblatt 82/22**

(45) Bekanntmachung des Hinweises auf die
Patenterteilung :
**02.10.85 Patentblatt 85/40**

(45) Bekanntmachung des Hinweises auf die
Entscheidung über den Einspruch :
**21.10.92 Patentblatt 92/43**

(84) Benannte Vertragsstaaten :
**DE FR GB SE**

(56) Entgegenhaltungen :
**FR-A- 2 158 806**
**US-A- 3 954 102**
**US-A- 3 987 789**

(56) Entgegenhaltungen :
**US-A- 4 066 073**
**US-A- 4 151 841**
**US-A- 4 187 839**
**Prospekt der Fa. American Medical Systems,
Inc., Minneapolis, Publication 01209: ˝Rear
Tip Extenders˝**

(73) Patentinhaber : **ESKA medical &
kunststofftechnik gmbh & co., vormals Walter
Koss
Industriestrasse 2 Postfach 1164
W-6222 Geisenheim am Rhein (DE)**

(72) Erfinder : **Koss, Walter,
Industriestrasse
D-6222 Geisenheim/Rhein (de)**

(74) Vertreter : **Blumbach Weser Bergen Kramer
Zwirner Hoffmann Patentanwälte
Sonnenberger Strasse 100
W-6200 Wiesbaden (DE)**

EP 0 052 858 B2

**Beschreibung**

Penisprothesen in Form eines biegsamen Stabes, der wenigstens im äußeren Bereich aus implantierbarem Kunststoff besteht, werden zur Behandlung der Impotenz bei neurologischen Erkrankungen, Gefäßerkrankungen, Verletzungen und in anderen Fällen jeweils paarweise in die Schwellkörper implantiert. Vorzugsweise besitzen diese Prothesen einen Kern, der beispielsweise aus Kunststoff oder auch aus verdrillten hochreinen Silberdrähten besteht, die beim Biegen nicht aufhärten. Nach dem Implantieren der biegsamen Stäbe kann der Penis in die jeweils gewünschte Form und Position gebogen werden. Solche Prothesen sind bekannt (DE-U-7 805 284).

Die bekannten Prothesen der vorstehend genannten oder ähnlichen Art haben sich in der Praxis vielfach bewährt. Es ergibt sich jedoch die Schwierigkeit, daß der Operateur zum implantieren jeweils in Anpassung an die anatomischen Verhältnisse des Patienten sowohl in der Dicke als auch insbesondere in der Länge genau passende Stäbe verwenden muß. In zwei oder sogar weiteren Durchmesserbereichen von beispielsweise 9,5 mm muß daher jeweils ein Sortiment von Stabpaaren mit beispielsweise sieben oder mehr verschiedenen Längen zur Verfügung stehen. Die Anschaffung eines solchen Sortiments ist so aufwendig, daß häufig eine entsprechende Anfangsinvestition aus finanziellen Gründen unterbleibt. Kompromisse in der richtigen Anpassung können aber zu nachteiligen Folgen für den Patienten führen.

Es ist auch bekannt (FR-A-2 158 806), einen aus einem Stück bestehenden, biegsamen Stab entsprechend den anatomischen Erfordernissen in mehrere Abschnitte unterschiedlicher Härte zu unterteilen, wobei eine Längenanpassung durch Abschneiden am proximalen Ende erfolgen kann. Ein einfaches Kürzen der Prothesenstäbe kurz vor der Implantation stößt jedoch auf Schwierigkeiten, weil unter den Bedingungen im Operationssaal und der gebotenen Eile keine saubere und glatte, gerundete äußere Form sichergestellt ist. Das gilt insbesondere auch dann, wenn die Prothesenstäbe einen inneren Kern aufweisen, weil dann entweder auch der Kern mitgekürzt werden müßte und demgemäß nicht mehr vom Kunststoff umschlossen ist, oder ein für das Kürzen vorgosehener Abschnitt des Stabes ohne Kern vorgesehen ist. Dann fehlt aber in diesem Abschnitt die innere Abstützung.

Es ist auch schon versucht worden, unterschiedliche Längen der Prothesenstäbe durch Anstecken von Abschnitten unterschiedlicher Länge zu verwirklichen. Der beim Zusammenstecken gebildete Spalt bringt jedoch beim Biegen die Gefahr mit sich, daß Gewebe eingeklemmt wird. Dadurch können Nekrosen und andere nachteilige Folgen entstehen.

Der Erfindung liegt demgemäß die Aufgabe zugrunde, eine implantierbare Penisprothese in Form eines biegsamen Stabs zu schaffen, die eine Längen- und/oder Durchmesseranpassung durch den Operateur ermöglicht. Die Lösung der Aufgabe ist im Patentanspruch 1 angegeben.

Nach dem Kürzen des Abschnitts ohne den Schlauchfortsatz je nach den anatomischen Verhältnissen kann dann vom Operateur vor der Implantation der Schlauchfortsatz aufgeschoben werden, wodurch einerseits der gegebenenfalls angeschnittene oder abgeschnittene Kern an seinem Ende verdeckt wird und andererseits kein Spalt entsteht, der beim Biegen die Gefahr des Einklemmens von Gewebeteilen mit sich bringt. Es hat sich gezeigt, daß bei richtiger Bemessung des Stabdurchmessers und Schlauchfortsatzes einige Zeit nach dem Aufschieben eine absolut dichte und mechanisch sehr feste Verbindung zwischen den Teilen zustande kommt. Ein Auseinanderziehen oder gegenseitiges Verdrehen ist kaum möglich. Die zusammengeführten Stäbe verhalten sich demgemäß sowohl beim Implantieren als auch nach dem Implantieren wie die einstückigen Stäbe. Die Wandstärke des Schlauchfortsatzes und gegebenenfalls der Durchmesser des anschließenden Stabes können abgewandelt werden, um gewünschte Dickenabmessungen der Stäbe zu erzielen. Beispielsweise führen Wandstärken des Schlauchfortsatzes zwischen 0,5 und 5 mm bei einem Innendurchmesser von 5 mm zu einem Gesamtdurchmesser zwischen 6 und 15 mm.

Weiterbildungen der Erfindung sind in den abhängigen Ansprüchen gekennzeichnet. So kann der mit dem Schlauchfortsatz versehene Abschnitt als kurze weiche Spitze ausgebildet sein und das distale Ende der Prothese bilden.

Wenn die Prothese in bekannter Weise einen Kern, der in wenigstens einem Abschnitt so ausgebildet ist, daß er in jeder durch Biegen erreichten Form verbleibt, und eine Hülle aus implantierbarem Material aufweist, besteht die Möglichkeit, daß bei dem mit dem Schlauchfortsatz versehenen Abschnitt ein Kern vorhanden ist, der in den Schlauchfortsatz hinein- oder über ihn hinausragt und daß der zugehörige andere Abschnitt eine zentrale Bohrung zur Aufnahme des Kerns besitzt. Dann ergibt sich ein durchlaufender Kern in beiden Abschnitten der Prothese, die sich demgemäß wie ein einstückiger Stab verhält. Auf das freie Ende des Kerns kann eine Kappe aufgesetzt werden, die insbesondere bei einem Kern aus verdrallten Drähten das Ende zusammenhält. Der Abschnitt mit der zentralen Bohrung kann so ausgebildet sein, daß er das distale oder auch das proximale Ende bildet. Darüber hinaus kann in Weiterbildung der Erfindung ein mittlerer Abschnitt auf beiden Seiten mit einem Schlauchfortsatz versehen sein und sowohl ein proximaler als auch ein distaler Abschnitt angesetzt werden, die je eine Bohrung zur Aufnahme des Kerns im mittleren Abschnitt aufweisen. Der Prothesenstab besteht dann aus drei Abschnitten, so daß eine Längenanpassung an mehreren Stellen erfolen kann.

Eine zusätzliche Weiterbildung sieht vor, daß der Stab einen weichen distalen Abschnitt, einen steifen mittleren Abschnitt und einen sehr steifen proximalen Abschnitt aufweist. Das kann beispielsweise durch eine entsprechende Bemessung des Kerns erreicht werden, wobei im weichen distalen Abschnitt der Kern auch ganz fehlen kann. Eine solche Unterteilung führt zu einer besseren Anpassung an die natürlichen Verhältnisse, wobei der steifere mittlere Abschnitt ein Abknicken verhindert und der sehr steife proximale Abschnitt eine gute Abstützung am Becken ermöglicht.

Es ist an sich erwünscht, daß das die Hülle bildende Kunststoffmaterial verhältnismäßig weich ist, damit die implantierten Stäbe nicht auffallen oder tastbar sind. Eine weiche Hülle bringt jedoch die Gefahr mit sich, daß der Penis beim Geschlechtsverkehr abknickt oder Drähte der Einlage austreten. Daher sieht eine Weiterbildung der Erfindung vor, daß die Hülle eine aus einem härteren Material bestehende, den Kern umgebende Innenschicht und eine aus einem weicheren Material bestehende Außenschicht aufweist. Es kann auch vorgesehen sein, daß statt der weicheren Außenschicht im radialen Abstand von der härteren Schicht auf den Kern eine Außenschicht angebracht ist, wobei in den Zwischenraum zwischen der Innen- und Außenschicht gegebenenfalls ein Kunststoffgel oder eine Flüssigkeit eingebracht werden kann.

Nachfolgend werden Ausführungsarten der Erfindung anhand der Zeichnungen beschrieben. Es zeigt

Fig. 1 ein Ausführungsbeispiel der Erfindung,

Fig. 2 ein weiteres Ausführungsbeispiel der Erfindung,

Fig. 3 ein drittes Ausführungsbeispiel der Erfindung,

Fig. 4, 5 und 6 Querschnittsansichten von drei verschiedenen Ausführungsbeispielen.

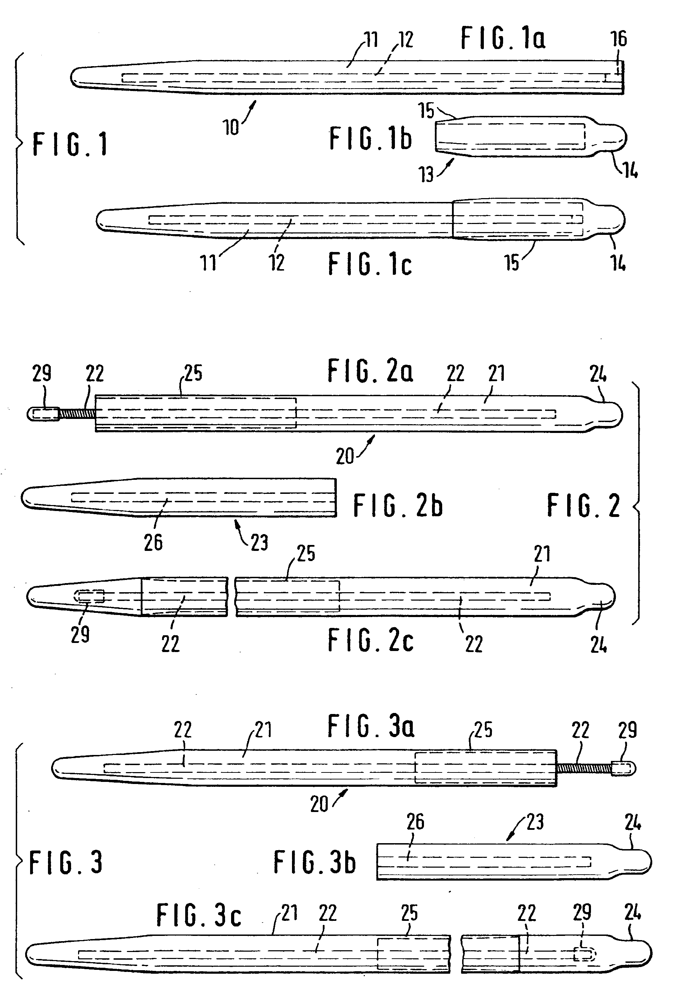

Fig. 1a und 1b zeigen zwei zusammensteckbare Abschnitte eines Prothesenstabes, der zusammengesteckt in Fig. 1c zu erkennen ist. Der längere Abschnitt 10 gemäß Fig. 1a weist eine Hülle 11 aus implantierbarem Kunststoff, beispielsweise Silikon oder einem anderen Elastomer, und einen eingebetteten Kern 12 auf, der beispielsweise aus verdrallten Silberdrähten besteht. Der kürzere Abschnitt 13, der später das distale Ende des Prothesenstabes bildet, besitzt eine weiche Spitze 14 mit einem anschließenden Schlauchfortsatz 15, die einstückig aus implantierbarem Kunststoff hergestellt sind. Der längere Abschnitt 10 kann auf der in der Darstellung rechten Seite auf die gewünschte Länge gekürzt werden. Danach wird der Schlauchfortsatz 15 gegebenenfalls nach Aufweitung mit einer Spreizzange und Anfeuchten mit destilliertem Wasser aufgeschoben.

Nach einiger Zeit bilden die beiden Abschnitte einen praktisch untrennbaren, biegsamen Stab gemäß Fig. 1c.

Um zu vermeiden, daß der Kern 12 nach dem Kürzen des Abschnitts 10 vorsteht, kann vor dem Schneiden des Kerns die Hülle 11 axial gestaucht werden, so daß nach dem Abschneiden des Kerns dessen Ende sich in einen kurzen Kanal 16 zurückzieht.

Beim Ausführungsbeispiel nach Fig. 2 ist wiederum in Fig. 2a der längere Abschnitt 20 des Prothesenstabs, in Fig. 2b der kürzere Abschnitt und in Fig. 2c der fertige Prothesenstab gezeigt. Der längere Abschnitt 20, der später das distale Ende der Prothese bildet, weist eine Hülle 21 aus Kunststoffmaterial mit einer weichen Spitze 24 am distalen Ende und einem Kern 22 ähnlich dem Kern 12 in Fig. 1 auf. Am proximalen Ende (links in Fig. 2) besitzt der Abschnitt 20 einen Schlauchfortsatz 25. Der Kern 22 steht aus dem Schlauchfortsatz 25 vor. Um zu vermeiden, daß die Drähte des Kerns sich lösen, ist auf das Ende eine Kappe 29, beispielsweise aus Teflon, aufgesteckt. Der kürzere Abschnitt 23 gemäß Fig. 2b besitzt eine zentrale Bohrung 26, die den freien Teil des Kerns 22 im Abschnitt 20 einschließlich der Kappe 29 aufnimmt, wie Fig. 2c zeigt. Der Abschnitt 20 kann mit unterschiedlichen Durchmessern und damit Wandstärken des Schlauchfortsatzes 25 gefertigt werden, so daß neben der Längenanpassung auch eine Dickenanpassung möglich ist.

Beim Kürzen der beiden Abschnitte 20, 23 einschließlich des Kerns 22 ist so vorzugehen, daß der Kern 22 mit der Kappe 29 die Bohrung 26 praktisch bis zum Ende ausfüllt, damit eine genügende Stützung erreicht wird.

Das Ausführungsbeispiel gemäß Fig. 3 entspricht im wesentlichen dem Ausführungsbeispiel gemäß Fig. 2. Es wurden lediglich die beiden Abschnitte vertauscht, d. h. es enthält jetzt der proximale Abschnitt 20 den Schlauchfortsatz 25 und der distale Abschnitt 23 die Bohrung 26 und die weiche Spitze 24.

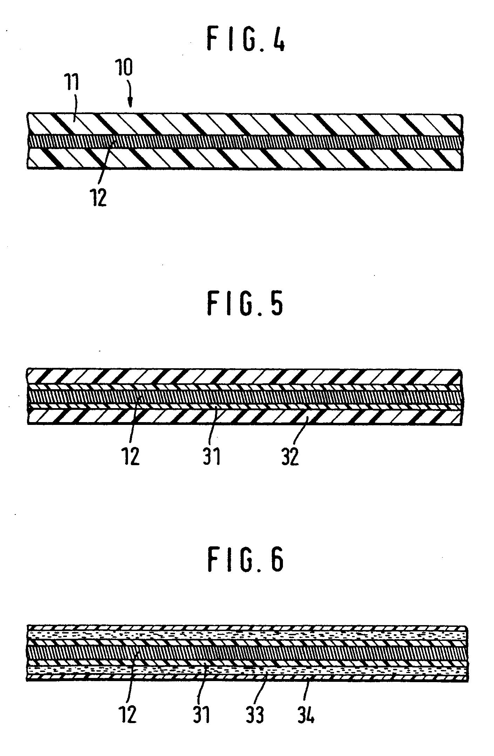

Fig. 4 zeigt schematisch einen Abschnitt eines Prothesenstabes, beispielsweise einen Teil des Stabes 10 gemäß Fig. 1. In der Kunststoffhülle 11 ist der Kern 12 angeordnet, der beispielsweise aus verdrallten Silberdrähten besteht. Fig. 5 zeigt eine vorteilhafte Abwandlung, bei der auf den Kern 12 zunächst eine Schicht 31 aus verhältnismäßig hartem Kunststoffmaterial, beispielsweise Silikon, aufgebracht ist. Diese Schicht wirkt als versteifende Schutzschicht, die ein Austreten des Kerns oder einzelner Drähte verhindert. Auf der inneren Schicht 31 ist eine äußere Schicht 32 aus verhältnismäßig weichem Kunststoffmaterial, beispielsweise Silikon, angeordnet, die dafür sorgt, daß der Stab sich leichter anpaßt und nicht tastbar ist.

Die weitere Variante gemäß Fig. 6 besitzt ebenfalls einen Kern 12 mit einer inneren Schutzschicht 31. Dann folgt radial auswärts ein mit einem Gel oder einer Flüssigkeit gefüllter Ringraum 33 und anschließend eine äußere Kunststoffschicht 34, die wiederum verhältnismäßig hart sein kann. Das Gel kann je nach Wunsch weicher oder härter eingestellt werden. Gegebenfalls kann der Ringraum 33 auch lediglich mit Luft gefüllt sein.

**Patentansprüche**

1. Penisprothese in Form eines biegsamen Stabes, der wenigstens im äußeren Bereich aus implantierbarem Kunststoff besteht und in wenigstens zwei Abschnitte unterteilt ist, dadurch gekennzeichnet, daß die Abschnitte als getrennte, zusammmensteckbare Abschnitte (10, 13; 20, 23) ausgebildet sind und daß an der bzw. den Stoßstellen der eine Abschnitt einen Schlauchfortsatz (15, 25) besitzt, der über den zur Längeneinstellung gekürzten anderen Abschnitt schiebbar ist und die durch das Kürzen entstandene Schnittfläche verdeckt.

2. Penisprothese nach Anspruch 1, dadurch gekennzeichnet, daß der mit dem Schlauchfortsatz (15) versehene Abschnitt (13) als kurze weiche Spitze (14) ausgebildet ist und das distale Ende der Prothese bildet.

3. Penisprothese nach Anspruch 1 oder 2, gekennzeichnet durch einen Kern (12, 22), der in wenigstens einem Abschnitt so ausgebildet ist, daß er in einer durch Biegen erreichten Form verbleibt, und eine Hülle (11, 21) aus implantierbarem Material.

4. Penisprothese nach Anspruch 3, dadurch gekennzeichnet, daß bei dem mit dem Schlauchfortsatz (25) versehenen Abschnitt (20) der Kern (22) in den Schlauchfortsatz hinein- oder über ihn hinausreicht und daß der zugehörige andere Abschnitt (23) eine zentrale Bohrung (26) zur Aufnahme des Kerns besitzt.

5. Penisprothese nach Anspruch 4, dadurch gekennzeichnet, daß auf das freie Ende des Kerns (22) eine Kappe (29) aufgesetzt ist.

6. Penisprothese nach Anspruch 4 oder 5, dadurch gekennzeichnet, daß der Abschnitt (23) mit der zentralen Bohrung (26) das distale Ende der Prothese bildet (Fig. 3).

7. Penisprothese nach Anspruch 4 oder 5, dadurch gekennzeichnet, daß der Abschnitt (23) mit der zentralen Bohrung (26) das proximale Ende der Prothese bildet (Fig. 2).

8. Penisprothese nach Anspruch 4 oder 5, dadurch gekennzeichnet, daß ein mittlerer Abschnitt auf beiden Seiten einen Schlauchfortsatz besitzt und ein proximaler und ein distaler Abschnitt je eine Bohrung zur Aufnahme des Kerns im mittleren Abschnitt aufweisen.

9. Penisprothese nach einem der Ansprüche 3 bis 8, dadurch gekennzeichnet, daß die Hülle eine aus einem härteren Material bestehende, den Kern (12) umgebende Innenschicht (31) und eine aus einem weicheren Material bestehende Außenschicht (32) aufweist (Fig. 5).

10. Penisprothese nach einem der Ansprüche 3 bis 8, dadurch gekennzeichnet, daß die Hülle eine aus einem härteren Material bestehende, den Kern umgebende Innenschicht (31) und eine im radialen Abstand angeordnete Außenschicht (34) aufweist.

11. Penisprothese nach Anspruch 10, dadurch gekennzeichnet, daß sich in dem Raum (33) zwischen der Innen- und der Außenschicht ein Kunststoffgel oder eine Flüssigkeit befindet.

12. Penisprothese nach einem der Ansprüche 1 bis 11, dadurch gekennzeichnet, daß der Stab einen weichen distalen Abschnitt, einen steiferen mittleren Abschnitt und einen sehr steifen proximalen Abschnitt aufweist.

**Claims**

1. A penis prosthesis in the form of a flexible rod made at least in its external region of an implantable plastics and subdivided into at least two parts, characterised in that the parts are separate parts (10, 13; 20, 23) adapted to be pushed together and at the or each junction one part has an extension (15, 25) in the form of a flexible hose which can be pushed over the other part shortened for length adjustment and which covers the cut surface resulting from the shortening.

2. A prosthesis according to claim 1, characterised in that the part (13) having the flexible tubular extension (15) is in the form of a short soft tip (14) and forms the distal end of the prosthesis.

3. A prosthesis according to claim 1 or 2, characterised by: a core (12, 22) so formed in at least one part as to remain in a shape produced by bending; and a tubular covering (11, 21) made of an implantable material.

**4.** A prosthesis according to claim 3, characterised in that in the case of the part (20) having the flexible tubular extension (25) the core (22) extends thereinto or therebeyond, and the associated other part (23) is formed with a central bore (26) to receive the core.

**5.** A prosthesis according to claim 4, characterised in that a cap (29) is placed on the free end of the core (22).

**6.** A prosthesis according to claim 4 or 5, characterised in that the part (23) which is formed with the central bore (26) forms the distal end of the prosthesis (Fig. 3).

**7.** A prosthesis according to claim 4 or 5, characterised in that the part (23) which is formed with the central bar (26) forms the proximal end of the prosthesis (Fig. 2).

**8.** A prosthesis according to claim 4 or 5, characterised in that a central part has an extension in the form of a flexible hose at both ends and a proximal part and a distal part are each formed with a bore to receive the core in the central part.

**9.** A prosthesis according to any of claims 3 to 8, characterised in that the tubular covering comprises: an inner layer (31) made of a relatively hard material and extending around the core (12); and a softer outer layer (32) (Fig. 5).

**10.** A prosthesis according to any of claims 3 to 8, characterised in that the tubular covering comprises: an inner layer (31) made of a relatively hard material and extending around the core; and an outer layer (34) disposed at a radial distance.

**11.** A prosthesis according to claim 10, characterised in that a plastics gel or a liquid is present in the space (33) between the inner layer and the outer layer.

**12.** A prosthesis according to any of claims 1 - 11, characterised in that the rod has a soft distal part, a more rigid central part and a very rigid proximal part.

**Revendications**

**1.** Prothèse de pénis se présentant sous la forme d'une tige souple qui, au moins dans sa zone extérieure est faite en matière plastique implantable et est divisée en au moins deux segments, caractérisée en ce que les segments sont des segments séparés (10, 13 ; 20, 23) pouvant être assemblés les uns aux autres, et qu'à l'emplacement du joint ou des joints, l'un des segments possède un prolongement tubulaire souple (15, 25) qui est enfilé par dessus l'autre segment raccourci pour détermination de la longueur et qui recouvre la surface de coupe résultant du raccourcissement.

**2.** Prothèse de pénis selon la revendication 1, caractérisée en ce que le segment (13) pourvu du prolongement tubulaire souple (15) a la forme d'une pointe (14) courte molle et forme l'extrémité distale de la prothèse.

**3.** Prothèse de pénis selon la revendication 1 ou 2, caractérisée par une âme (12, 22) qui, dans au moins l'un des segments, est agencée de façon à rester dans la forme obtenue par courbure, et par une enveloppe (11, 21) en matériau implantable.

**4.** Prothèse de pénis selon la revendication 3, caractérisée en ce que, dans le segment (20) pourvu du prolongement tubulaire souple (25), l'âme s'engage à l'intérieur du prolongement tubulaire souple et fait saillie au-delà de celui-ci, et que l'autre segment (23) correspondant possède un évidement (26) central pour recevoir l'âme.

**5.** Prothèse de pénis selon la revendication 4, caractérisée en ce qu'un capuchon (29) est posé sur l'extrémité libre de l'âme (22).

**6.** Prothèse de pénis selon la revendication 4 ou 5, caractérisée en ce que le segment (23) pourvu de l'évidement (26) central forme l'extrémité distale de la prothèse (figure 3).

**7.** Prothèse de pénis selon la revendication 4 ou 5, caractérisée en ce que le segment (23) pourvu de l'évidement (26) central forme l'extrémité proximale de la prothèse (figure 2).

**8.** Prothèse de pénis selon la revendication 4 ou 5, caractérisée en ce qu'un segment médian possède des deux côtés un prolongement tubulaire souple et en ce qu'un segment proximal et un segment distal présentent chacun un évidement central pour recevoir l'âme qui se trouve dans le segment médian.

**9.** Prothèse de pénis selon une quelconque des revendications 3 à 8, caractérisée en ce que l'enveloppe comporte une couche intérieure (31) faite en matériau dur, entourant l'âme (12) et une couche extérieure (32) faite en matériau mou (figure 5).

**10.** Prothèse de pénis selon une quelconque des revendications 3 à 8, caractérisée en ce que l'enveloppe comporte une couche intérieure (31) faite en matériau dur entourant l'âme et une couche extérieure (34) disposée à une certaine distance dans le sens radial.

**11.** Prothèse de pénis selon la revendication 10, caractérisée en ce qu'un gel plastique ou un liquide se trouve dans l'espace (33) entre la couche intérieure et la couche extérieure.

**12.** Prothèse de pénis selon une quelconque des revendications 1 à 11, caractérisée en ce que la tige comporte un segment distal mou, un segment médian rigide et un segment proximal très rigide.

FIG.1a
FIG.1b
FIG.1c
FIG.1

FIG.2a
FIG.2b
FIG.2c
FIG.2

FIG.3a
FIG.3b
FIG.3c
FIG.3

# FIG. 4

# FIG. 5

# FIG. 6